**Europäisches Patentamt**

**European Patent Office**

**Office européen des brevets**

(19)

(11) Publication number : **0 526 225 A1**

# EUROPEAN PATENT APPLICATION

(21) Application number : **92306984.3**

(22) Date of filing : **31.07.92**

(51) Int. Cl.⁵ : **A61F 13/15**

(30) Priority : **02.08.91 US 739935**

(43) Date of publication of application :
**03.02.93 Bulletin 93/05**

(84) Designated Contracting States :
**AT BE CH DE DK ES FR GB IT LI LU MC NL PT SE**

(71) Applicant : **JOHNSON & JOHNSON INC.**
**2155 Boulevard Pie IX**
**Montreal, Quebec H1V 2E4 (CA)**

(72) Inventor : **Murji, Zulfikar**
**1850 Bercy St.App., No. 110**
**Montreal, Quebec, H2K QU2 (CA)**
Inventor : **Ramacieri, Patricia**
**11640 Alfred Laurence**
**Montreal, H1E 3T4 (CA)**

(74) Representative : **Mercer, Christopher Paul et al**
**Carpmaels & Ransford 43, Bloomsbury Square**
**London WC1A 2RA (GB)**

(54) Hygenic disposable absorbent product.

(57) A disposable absorbent product, such as a sanitary napkin (10), a diaper, an adult brief, an incontinence pad or a wound dressing, comprising an ultra-thin absorbent core (22) and a bulk layer of filler material (32) adjacent the ultra-thin absorbent core, giving the illusion of an absorbent product with a thick absorbent pad, in order to provide a certain degree of mental comfort and security to a wearer with a skeptical attitude toward ultra-thin absorbent products. The invention also extends to a method for enhancing the user acceptance of a disposable absorbent product with an ultra-thin absorbent core by providing in the disposable absorbent product a bulk layer of filler material.

Fig.9

Fig.8

EP 0 526 225 A1

The invention relates to the art of manufacturing disposable absorbent products, more particularly, to a disposable absorbent product having an ultra-thin absorbent core and a bulk layer of filler material giving the illusion of a thick absorbent product, in order to provide a certain degree of mental comfort and security to a wearer with a skeptical attitude toward ultra-thin absorbent products. The invention also extends to a method for enhancing the public acceptance of a disposable absorbent product with an ultra-thin absorbent core.

Traditionally, sanitary napkins for medium to high menstrual flow have been relatively thick and bulky containing an absorbent core made of fluffed hydrophilic materials such as wood pulp, rayon or cotton. These absorbent materials are attractive because they are fairly easy to manufacture at low cost while having, at least theoretically, a high absorbent capacity. However, this category of disposable absorbent products are known to possess severe deficiencies, failing in practice to provide the required protection against leakage and staining. It has been shown that a thick absorbent core does not necessarily provide the required absorbency and protection in use, since the fibrous materials currently employed suffer from poor fluid distribution properties and do not have the ability to disperse fluid within the entire available absorbent volume. As a result, absorbed fluid will tend to remain localized to a certain area of the absorbent core, causing saturation and collapse in that area. This phenomenon often results in fluid overflow and leakage.

Presently available thick absorbent cores have a poor mechanical stability, tending to bunch-up and twist easily under the effect of compressive forces exerted by the wearer's thighs and pudendal region. The resultant distortions reduce the surface area available for the absorption of fluid, often resulting in a premature pad failure.

Another important disadvantage of sanitary napkins with thick absorbent cores resides in their inability to closely conform and follow the contour of the human body, achieving at best an intermittent contact surface between the fluid permeable side of the sanitary napkin and the skin or integument of the wearer, which results in poor gasketing increasing the likelihood of fluid leaking past the edges of the sanitary napkin and staining the wearer's clothes.

In addition, thick sanitary napkins cause a high degree of wearing awareness in terms of the "feel" of the sanitary napkin and the lack of product discretion, i.e. obtrusive pads discernible through the woman's apparel.

To address the above-discussed problems, a growing number of thinner and more flexible sanitary napking have been developed over the past recent years, providing better fit, comfort and discretion, while being sufficiently absorbent to provide an effective menstrual protection. Absorbent cores used in ultra-thin sanitary napkins include compressed sphagnum peat moss which is a highly dense absorbent material having a considerable fluid retaining capacity, as well as excellent fluid wicking and drying power. Other ultra-thin absorbent cores are laminates, including hydrogel-forming, polymer gelling agents, also known in the trade as "super-absorbents", providing enhanced absorbent capacity as well as improved fluid retention performance. Yet, more recent developments of thin, flexible and efficient absorbent cores employ debonded pulp boards that have been mechanically tenderized, as well as densified cellulosic pulp fluff sheets that have been subjected to a perfembossing treatment. Generally speaking, these ultra-thin absorbent cores have very good fluid absorption, fluid retention and wicking characteristics in addition to providing a high structural integrity and good comfort potential.

Sanitary napkins with ultra-thin absorbent cores claiming to provide exceptional menstrual protection are presently making entries on the market, offering discretion and comfort as well as an effective menstral protection. However, there is a high degree of skepticism in certain consumer groups that ultra-thin sanitary napkins have the absorbency required for medium to heavy menstrual flows. These consumer groups have a tendency to associate thickness with absorbercy and are not likely to trust an ultra-thin sanitary napkin to match in terms of protection a traditional thick pad.

To a certain extent, this mental conditioning is attributable to supply and marketing of conventional sanitary napkins by following a scheme of product classification linking thickness with absorbency requirement; i.e. the sanitary napkins for heavy menstrual days and overnight protection being considerably thicker than sanitary napkins for the end of the menstral period, referred in the trade as "mini pads". In reality, the extra absorbent material in thicker sanitary napkins is not necessarily fully utilized due to the poor fluid dispersion characteristics of conventional absorbent cores, therefore the extra amount of absorbent material does not necessarily provide added benefits over thinner sanitary napkins.

Hence, there continues to exist a consumer need for a sanitary napkin providing an improved leakage protection and also giving to the wearer the mental comfort and security of a thick traditional sanitary napkin. In all likelihood, as more and more consumers experience the benefits of ultra-thin sanitary napkins, trust will emerge toward this product, especially when the benefits in terms of leakage protection, comfort and discretion, become apparent. However, the conversion may be gradual and there may be consumers who will always prefer a thicker sanitary napkin.

An object of the present invention is to provide a disposable absorbent product with an ultra-thin absorbent core, capable to provide the mental comfort and security of an absorbent product with a thicker and bulkier absorbent core.

Another object of the invention is to provide a method for enhancing user acceptance of a disposable absorbent product with an ultra-thin absorbent core by building in the absorbent product a psychological assurance factor providing the mental comfort and security of an absorbent product with a thick absorbent core.

The present inventors have made the unexpected discovery that by providing into a disposable absorbent product such as a sanitary napkin, a diaper, an adult brief, an incontinence pad, or a wound dressing employing an ultra-thin absorbent core, a bulk layer of filler material giving the visual and tactile impression of an absorbent product with a thick and bulky absorbent core, the psychological need of consumers, skeptical of ultra-thin absorbent products, is fulfilled, while providing the benefits and advantages of modern ultra-thin absorbent cores, such as good absorbency, high degree of leakage protection and excellent comfort.

In one aspect, the invention provides a disposable absorbent product comprising an ultra-thin, functional absorbent core and a bulk layer juxtaposed to the ultra-thin, functional absorbent core, the bulk layer constituting means to provide the illusion of a disposable absorbent product with an absorbent core considerably thicker than the ultra-thin, functional absorbent core.

As embodied and broadly described herein, the invention further comprises a method for enhancing user acceptance of a disposable absorbent product having an ultra-thin, functional absorbent core, comprising the step of juxtaposing to the ultra-thin, functional absorbent core a bulk layer of filler material providing the illusion of a disposable absorbent product with an absorbent core considerably thicker than the ultra-thin, functional absorbent core.

In a preferred embodiment, the disposable absorbent product comprises an envelope with a fluid-permeable cover layer and a fluid-impervious backing layer, enclosing the absorbent/illusory system. Since the bulk layer is not intended to be involved in the fluid absorption process of the absorbent product, it is located between the ultra-thin, functional absorbent core and the fluid-impervious backing layer, thus being remote from the main fluid path, extending from the fluid-permeable cover layer to the ultra-thin, functional absorbent core.

Preferably, the bulk layer of filler material is highly flexible to preserve unimpaired the comfort and the superior gasketing characteristics of an ultra-thin absorbent product. To this end, the bulk layer has a density considerably less than the density of the ultra-thin, functional absorbent core. It should be noted that lowering the density of the bulk layer beyond a certain point may not necessarily be desirable because the wearer looses the tactile impression of a thick pad. Ideally, the bulk layer should provide the visual impression and the feel of a thick pad while preserving the advantages of ultra-thin absorbing products. In preferred embodiments. the density of the bulk layer is in the range of from about 0.01 to about 0.1 grams per cubic centimeter (g/cc), more preferably of from about 0.02 to about 0.06 g/cc and most preferably of from about 0.03 to about 0.05 g/cc. By comparison, the density of a typical, ultra-thin absorbent core (in the case of an absorbent system transfer layer/ reservoir layer, the transfer layer is excluded) is considerably higher, preferably in the range of from about 0.1 to about 1.0 g/cc, more preferably in the range of from about 0.2 to 0.6 g/cc.

The lower density of the bulk layer provides a very poor wicking power index, whereby fluid is inhibited from migrating under the effect of capillary action from the ultra-thin, functional absorbent core to the bulk layer. The high density differential does not, however, entirely preclude fluid entering the bulk layer. Under some particular circumstances, such as when the ultra-thin, functional absorbent core is fully saturated, or large compressive forces are exerted on the disposable absorbent product, a limited fluid transfer may occur. This is not necessarily undesirable because in extreme conditions, the bulk layer may assist the ultra-thin, functional absorbent core to absorb large quantities of fluid by providing an additional absorbent volume. It should be noted, however, that any benefit derived from the limited capacity of the bulk layer to take-up fluid is merely incidental to its primary function which is to provide the illusion of a thick pad.

The dry thickness of the bulk layer dictates the degree of mental comfort and security provided by the disposable absorbent product. In practice, this parameter is carefully selected according to the characteristics of the consumer group which is being targeted. The bulk layer has preferably a dry thickness in the range of from about 0.1 to about 1.0 inches measured at 0.05 pounds per square inch (psi) pressure, more preferably in the range of from about 0.25 to about 0.75 inches measured at 0.05 psi pressure and most preferably in the range of from about 0.30 to about 0.60 inches measured at 0.05 psi pressure. By comparison, the dry thickness of an ultra-thin absorbent core (in case of an absorbent system transfer layer/reservoir layer, the transfer layer is excluded) is preferably in the range of from about 0.01 to 0.15 inches measured at 0.05 psi pressure, more preferably in the range of from about 0.025 to about 0.075 inches measured at 0.05 psi pressure.

It has been found advantageous to emboss a decorative pattern on the fluid absorbent surface of the disposable absorbent product to slightly compress and density the bulk layer to flatten large irregularities thereon, which otherwise may reveal the artificially padded nature of the disposable absorbent product. In addition, the embossing operation also allows to control the thickness of the disposable absorbent product and to enhance its stability. As a result of the embossing operation, the surface of the disposable absorbent product is overall flatter, albeit locally it displays irregularities due to the embossing and it is also firmer, providing the tactile impression of a traditional thick absorbent core. If a decorative pattern is not required, the disposable absorbent product may be treated with compression only.

In preferred embodiments, the dry thickness of the disposable, absorbent product after embossing is in the range of from about 0.2 to about 1.0 inch measured at 0.05 psi pressure, more preferably of from about 0.33 to about 0.63 inches at 0.05 psi pressure. These values include the average thickness of a fluid-permeable cover layer and of a fluid-impervious backing layer which sandwich the absorbent/illusory system.

As embodied and broadly described herein, the invention also provides a disposable absorbent product, comprising:

- a top fluid-permeable cover layer;
- a bottom fluid-impervious backing layer;
- an ultra-thin, functional absorbent core between said layers; and
- a bulk layer of filler material between said ultra-thin, functional absorbent core and said fluid-impervious backing layer, said ultra-thin, functional absorbent core having a density substantially higher than the density of said bulk layer which hinders fluid migration from said ultra-thin, functional absorbent core toward said bulk layer under the effect of capillary action whereby the fluid has greater affinity for the denser layer. The density of said bulk layer is selected to enhance the tactile sensation provided by said disposable absorbent product when worn, giving to a wearer a feel of an absorbent product employing an absorbent core considerably bulkier than said ultra-thin, functional absorbent core. Further, the bulk layer is considerably thicker than said ultra-thin, functional absorbent core, giving a visual impression of an absorbent product with an absorbent core considerably thicker than said ultra-thin, functional absorbent core whereby the bulk layer provides an increased degree of mental comfort and security to a wearer with a skeptical attitude or unfavorable predisposition toward ultra-thin absorbent products.

As embodied and broadly described herein, the invention provides a method for enhancing user acceptance of a disposable absorbent product comprising:

- a top fluid-permeable cover layer;
- a bottom fluid-impervious backing layer;
- an ultra-thin, functional absorbent core between said layers, said method comprising the step of providing between said ultra-thin, functional absorbent core and said fluid-impervious backing layer, a bulk layer having a density considerably lower than the density of said ultra-thin, functional absorbent core which hinders fluid migration from said ultra-thin, functional absorbent core toward said bulk layer under the effect of capillary action whereby fluid has a greater affinity for the denser layer. The density of said bulk layer is selected to enhance the tactile sensation provided by said disposable absorbent product when worn, giving to a wearer a feel of an absorbent product having an absorbent core considerably bulkier than said ultra-thin, functional absorbent core. Further, the bulk layer is considerably thicker than said ultra-thin, functional absorbent core, giving a visual impression of an absorbent product with an absorbent core considerably thicker than said ultra-thin, functional absorbent core whereby said bulk layer provides an increased degree of mental comfort and security to a wearer with a skeptical attitude or unfavourable predisposition toward ultra-thin absorbent products.

The bulk layer also provides a certain degree of added resiliency and stability to the disposable absorbent product. The resiliency factor provides a mechanical "memory" to the absorbent product. The ability of the bulk layer in a strained condition, by virtue of a relatively high yield strength to recover its form after deformation, allows the disposable absorbent product to return in intimate contact against the integument of the wearer after being compressed, thus maintaining good gasketing regardless of the posture of the wearer.

The stability of the bulk layer provides an improved mechanical resistance to the absorbent product, preventing a loss of structural integrity in use. Although an ultra-thin absorbent core is already highly resistant, taking advantage of the bulk layer to enhance the resistance of the absorbent product further contributes to reduce failures due to tearing, breakage or other mechanical damage of the absorbent product in use.

To further enhance the stability of the disposable absorbent product, the bulk layer and the ultra-thin absorbent core may be bonded to one another.

Suitable filler materials for manufacturing the bulk layer are soft fibers, such as wood pulp fluff, cotton and rayon. Synthetic thermoplastic fibers, or wood pulp-synthetic fiber blends of polyethylene, polyester or polypropylene are preferred when it is desired to provide resiliency and stability to the bulk layer.

As embodied and broadly described herein, the invention provides a disposable absorbent product, comprising an ultra-thin, functional absorbent core and a bulk layer having a non-uniform thickness juxtaposed to said ultra-thin, functional absorbent core, said bulk layer constituting means to provide a visual and a tactile impression of a disposable absorbent product with an absorbent core considerably thicker than said ultra-thin, functional absorbent core, having a raised area at a selected location on the disposable absorbent product.

As embodied and broadly described herein, the invention provides a method for enhancing user acceptance of a disposable absorbent product having an ultra-thin, functional absorbent core, comprising the step of juxtaposing to said ultra-thin, functional absorbent core a bulk layer of filler material having a non-uniform thickness to provide the illusion of a disposable absorbent product with an absorbent core considerably thicker than said ultra-thin, functional absorbent core, having a raised area at a selected location on the disposable absorbent product.

In one embodiment, the bulk layer is shaped to provide a raised area near or at the central region of the disposable absorbent product, giving the impression to the wearer of a better absorbency potential in this area. The bulk layer may be sculptured as desired to provide the desired illusory absorbency profile to the disposable absorbent product.

In another embodiment, the raised area is provided along the edge portions of the disposable absorbent product to give the illusion of an absorbent core with a recessed area functioning as a reservoir to contain a coherent mass of body fluid.

As embodied and broadly described herein, the invention provides a disposable absorbent product, comprising an ultra-thin, functional absorbent core and a bulk layer juxtaposed to said ultra-thin, functional absorbent core, said bulk layer being thicker than said ultra-thin, functional absorbent core and being in a partially co-extensive relationship therewith, said bulk layer constituting means to provide a visual and a tactile impression of a disposable absorbent product having a raised area at a selected location on the disposable absorbent product.

As embodied and broadly described herein, the invention provides a method for enhancing user acceptance of a disposable absorbent product having an ultra-thin, functional absorbent core, comprising the step of juxtaposing to said ultra-thin, functional absorbent core a bulk layer, said bulk layer being thicker than said ultra-thin, functional absorbent core and being in a partially co-extensive relationship therewith to provide the illusion of a disposable absorbent product with an absorbent core having a raised area at a selected location on the disposable absorbent product.

This aspect of the invention is particularly useful for applications where it is desired to provide a psychological comfort factor only to a certain area of the disposable absorbent product. The dimensions and the specific location of the bulk layer on the ultra-thin, functional absorbent core is selected as desired. In a preferred embodiment, the bulk layer is a padded insert centrally located on the ultra-thin, functional absorbent core to provide a raised area only in that zone.

In the accompanying drawings
- Figure 1 is a fragmentary perspective view of a sanitary napkin constructed in accordance with the present invention;
- Figure 2 is a side elevational view of the sanitary napkin shown in Figure 1;
- Figure 3 is a cross-sectional view taken along lines 3-3 in Figure 2;
- Figure 4 is a fragmentary perspective view of a sanitary napkin, constructed in accordance to a first variant;
- Figure 5 is a side elevational view of the sanitary napkin shown in Figure 4;
- Figure 6 is a cross-sectional view taken along lines 6-6 in Figure 5;
- Figure 7 is a fragmentary perspective view of a sanitary napkin according to a second variant;
- Figure 8 is a side elevational view of the sanitary napkin shown in Figure 7;
- Figure 9 is a cross-sectional view along lines 9-9 in Figure 8;
- Figure 10 is a fragmentary perspective view of a sanitary napkin according to a third variant;
- Figure 11 is a side elevational view of the sanitary napkin shown in Figure 10;
- Figure 12 is a cross-sectional view along lines 12-12 in Figure 11; and
- Figure 13 is a perspective view of a sanitary napkin constructed in accordance with a fourth variant.

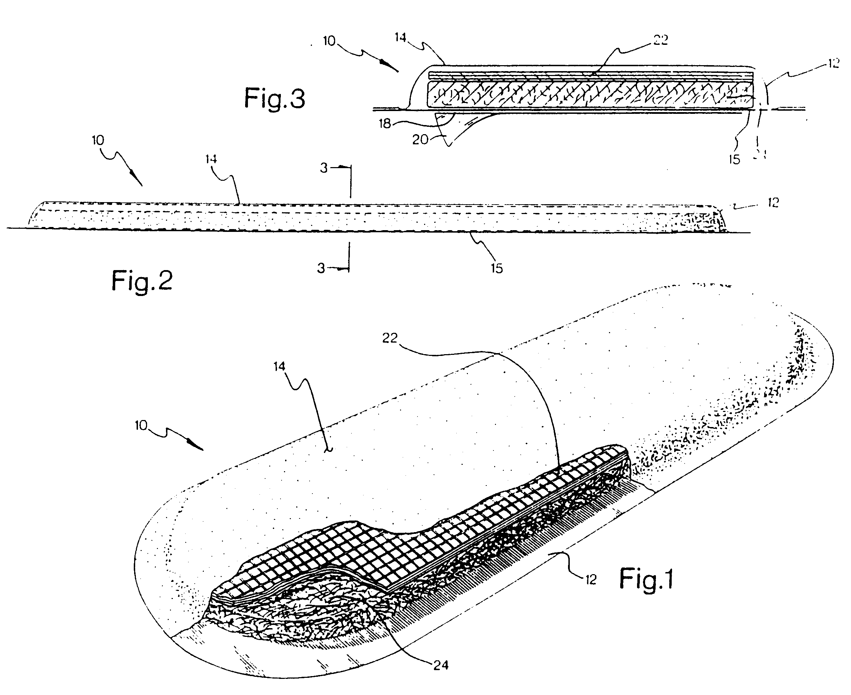

Figure 1 illustrates a sanitary napkin which embodies the concept of a disposable absorbent product with an ultra-thin absorbent core and a bulk layer of filler material providing the illusion of a thick absorbent pad.

The sanitary napkin, designated comprehensively by the reference numeral 10 comprises an envelope 12 defining an internal space receiving the absorbent/illusory structure of the sanitary napkin 10. The envelope 12 includes a fluid-permeable cover layer 14 made of nonwoven fabric or any other suitable porous web or apertured film, and a fluid-impervious backing layer 15, made of polyethylene film for example. The cover and backing layers 14 and 15 are heat-sealed to one another along their marginal portions.

To attach the sanitary napkin 10 to the wearer's underpants, the fluid-impervious backing layer 15 is provided with adhesive coated zone 18, covered with a pealable backing 20 to be removed before installing the sanitary napkin 10 in place.

In the internal space defined by the envelope 12 of the sanitary napkin 10, is placed an ultra-thin absorbent core 22 to absorb body fluid discharged on the fluid-permeable cover layer 14. The ultra-thin absorbent core 22 may be a thin sheet of compressed peat-moss material, a debonded, perf-embossed pulp board or a compressed, perf-embossed cellulosic pulp fluff sheet, among others. The absorbent core 22 may also be a laminated structure, comprising for example, a fluid transfer layer and a reservoir layer. In addition, a laminate of super-absorbent polymer materials may be incorporated in the ultra-thin absorbent core.

Below the ultra-thin absorbent core 22 is provided a bulk layer 24 made of filler material whose primary function is to give a visual and a tactile impression of a thick and bulky absorbent pad providing a psychological feeling of security to users being skeptical of ultra-thin sanitary napkins (for medium to heavy flow menstrual usage).

For a better stability of the sanitary napkin 10, the ultra-thin absorbent core 22 and the bulk layer 24 are bonded to one another by any suitable means.

Suitable filler materials for manufacturing the bulk layer 24 are soft fibers, such as wood pulp fluff, cotton and rayon. Synthetic thermoplastic fibers, or wood pulp-synthetic fiber blends of polyethylene, polyester or polypropylene, may also be used, which in addition to the bulk factor also provide added stability and increased pad resilience and integrity.

Since the bulk layer 24 is made of fibers acting as filler material, residue materials (recycled waste) from manufacturing machines of sanitary napkins and incontinence products or other related health-care products could be used, making the bulk layer 24 very economical to manufacture. For example, the residue material could be recycled into grinding mills for making pulp fluff and be mixed with pulp fluff at different ratios to make the bulk layer 24 at the desired stability and resiliency. This manufacturing technique also would yield more "environmentally friendly" products as a result of the reduction of solid manufacturing residue. It should also be appreciated that the fiberized material used in the bulk layer 24 does not require any aesthetical quality since it is covered by the ultra-thin absorbent core 22.

The thickness of the bulk layer 24 is an important factor as it influences the degree of mental comfort provided by the sanitary napkin; the thicker the bulk layer 24, the more secure the wearer will feel. This parameter can vary within wide limits, however, in order to have a significant effect on the public acceptance of the product, it should be at least as thick as the ultra-thin absorbent core 22.

Since the bulk layer 24 is not intended to interfere with the fluid absorption process of the sanitary napkin 10 nor with its comfort factor, the structure and location of the bulk layer 24 should be such as to preserve unimpaired the utilitarian functions of the sanitary napkin, although a wearer should be able to feel in use the added thickness giving a sense of security. To this end, the bulk layer 24 is located under the ultra-thin absorbent core 22, to remain remote from the main fluid bath in the sanitary napkin 10, from the fluid permeable cover layer 14 to the ultra-thin absorbent core 22. In addition, the filler material constituting the bulk layer 24 has a density significantly less than the density of the ultra-thin, functional absorbent core 22, selected to prevent a significant increase in the rigidity of the sanitary napkin which may result into a comfort reduction and poor gasketing, i.e. the inability of the sanitary napkin to conform to the body, while enhancing the tactile sensation provided by the sanitary napkin 10, giving the user the feel of a thick pad.

The lateral flexibility and the tensile strength of the bulk layer 24 are selected to provide the desired resiliency and stability characteristics required. These parameters are determined mainly from the formulation of the fiber constituent of the bulk layer.

It has been found advantageous to emboss the sanitary napkin 10 for creating a decorative pattern on the cover layer 14. The embossing process is carried out in a conventional manner, by passing the sanitary napkin 10 between two polls (not shown in the drawings), the roll which is in contact with the cover layer 14 having a surface sculptured to produce the desired pattern on the sanitary mapkin 10. The embossing operation also densifies and flattens to some degree the bulk layer 24 providing the sanitary napkin 10 with a generally flat fluid absorbent surface, substantially free of large distortions, albeit locally distortions exist due to the embossing, and which also becomes relatively firm. When a decorative pattern is not desired, the sanitary napkin 10 may be subjected to compression only by using smooth surfaced rolls.

The following Table provides an example of the density and thickness ranges for the bulk layer 24 and for the absorbent core 22, and a thickness range for the sanitary napkin 10. These ranges for the thickness and density parameters have been found optimal, providing good comfort and the illusion of a thick, traditional absorbent pad.

**TABLE**

| | Bulk Layer<br>(wood pulp fluff) | Ultra-Thin Absorbent Core *<br>core (peat moss material) | Sanitary<br>napkin ** |
|---|---|---|---|
| DENSITY (g/cc) | 0.03-0.05 | 0.2-0.6 | - - - |
| DRY THICKNESS (inches) | 0.30-0.60 | 0.025-0.075 | 0.33-0.63 |

\* For an absorbent core having a transfer layer and a reservoir layer, the density values are for the reservoir layer alone.

\** The thickness range takes in consideration the average thickness of the cover layer 14 and of the backing layer 15.

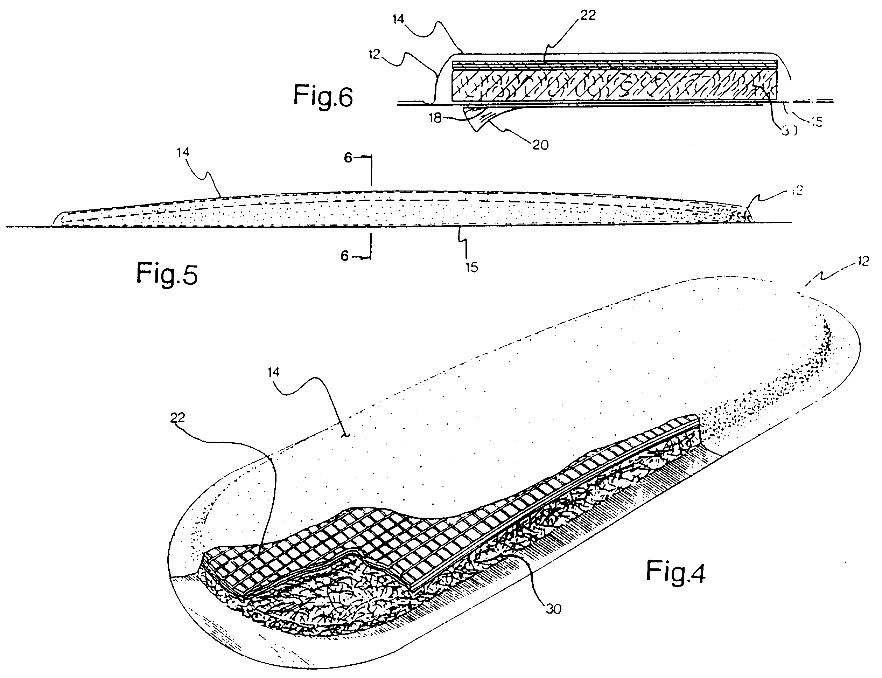

Figures 4, 5 and 6 illustrate a first variant of the sanitary napkin according to the invention. The salient point of this form of construction resides in the use of a non-planar bulk layer 30 providing the visual and tactile impression of a disposable absorbent product with a sculptured absorbent core having a raised portion at the center and giving the illusion of an enhanced absorbency potential in this area.

More specifically, the bulk layer 30 displays two oblique surfaces, oriented longitudinally and merging along an apex line which extends transversely to the sanitary napkin. This configuration gives the impression of an absorbent core thicker at the center and tapering toward its longitudinal end portions. The thinner end portions provide a better comfort and product discretion.

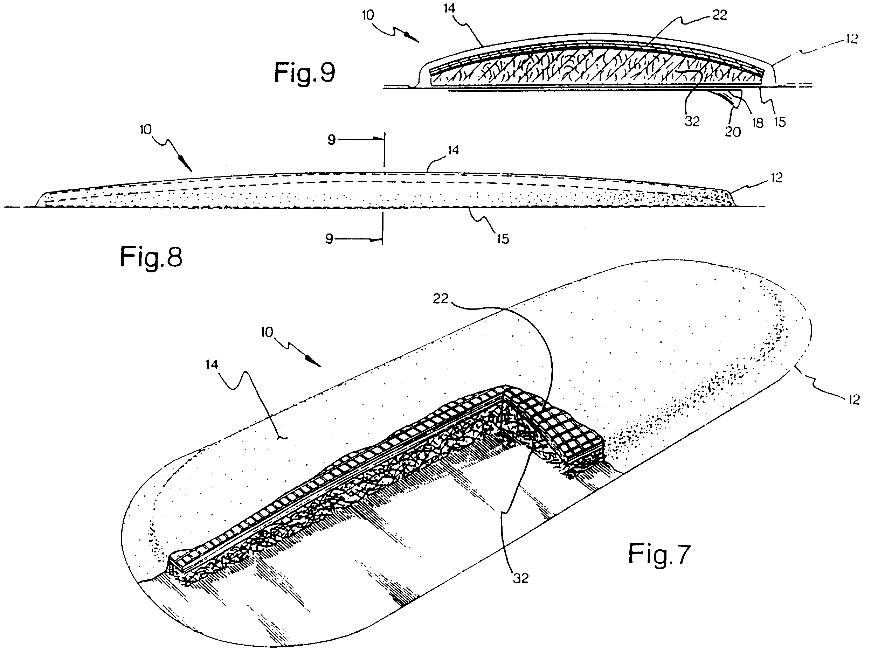

A further variant is depicted in Figures 7, 8 and 9. In this embodiment, the bulk layer 32 in addition to tapering toward the longitudinal end portions of the sanitary napkin also tapers toward its side edges. This form of construction provides a raised area culminating at a narrow apex located exactly in the center of the sanitary napkin.

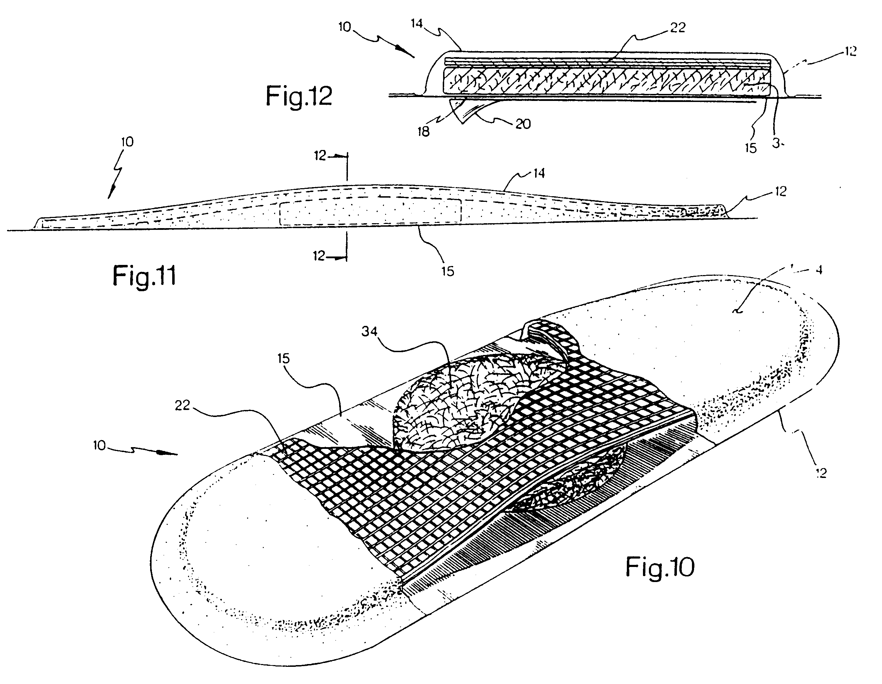

In a further variant shown in Figures 10, 11 and 12, the bulk layer 34 covers only a portion of the ultra thin, functional absorbent core 22 to provide the illusion of an absorbent core thicker only at a selected region of the disposable absorbent product, and thin elsewhere. More specifically, the bulk layer 34 is disc-shaped, it is about three times as thick as the ultra-thin, functional absorbent core, providing the impression of a sanitary napkin with a raised central area. This form of construction is well-suited for applications which require only a minimal psychological comfort factor, or where it is desired to maintain the appearance of an ultra-thin disposable absorbent product and simulate a variable absorbency potential.

The embodiments illustrated in Figures 4 to 12, are designed to provide the illusion of a better absorbency potential at the center of the sanitary napkin, where logically it should be located because the fluid impact point is likely to be proximal. Mowever, the invention is not restricted to this characteristic. It may very well be envisaged to locate the raised area elsewhere.

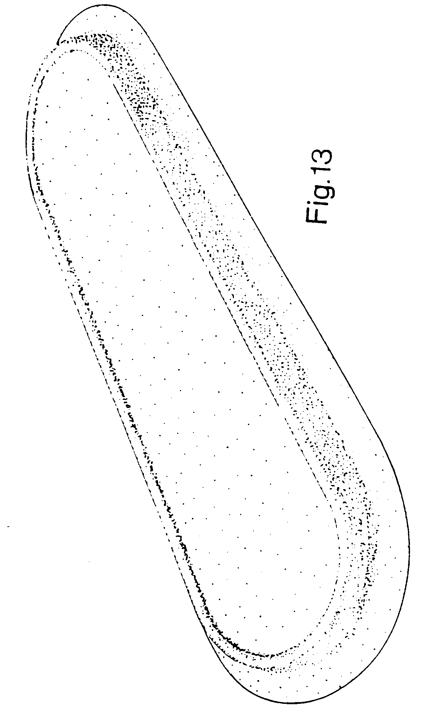

For example, as depicted in Figure 13, the raised area extends along the peripheral portion of the sanitary napkin providing the illusion of an absorbent core with a recessed center, functioning as a reservoir with the ability to contain a coherent body of fluid.

The scope of the present invention is not limited by the description, examples and suggestive uses herein and modifications can be made without departing from the spirit of the invention. Applications of the product and methods of the present invention for sanitary and other health-care uses can be accomplished by any sanitary protection, incontinence, medical, and absorbent methods and techniques as are presently or prospectively known to those skilled in the art. Thus, it is intended that the present application covers the modifications and variations of this invention provided that they come within the scope of the appended claims and their equivalents.

## Claims

1. A disposable absorbent product, comprising an ultra-thin, functional absorbent core and a bulk layer juxtaposed to said ultra-thin, functional absorbent core, said bulk layer constituting means to provide the illusion of a disposable absorbent product with an absorbent core considerably thicker than said ultra-thin, functional absorbent core.

2. The product of claim 1, which is a sanitary napkin, diaper, adult briefs, incontinence pad or wound dressing.

3. The product of claim 1 or claim 2, wherein said ultra-thin, functional absorbent core has a dry thickness in the range of from about 0.01 to about 0.15 inches, preferably about 0.025 to about 0.075 inches, measured at 0.05 psi pressure.

4. The product of any one of claims 1 to 3, wherein said ultra-thin, functional absorbent core has a density in the range of from about 0.1 to about 1.0 g/cc, preferably from about 0.2 to about 0.6 g/cc.

5. The product of any one of claims 1 to 4, wherein said bulk layer has a dry thickness in the range of from about 0.1 to about 1.0 inches, preferably 0.25 to 0.75 inches, most preferably 0.30 to 0.60 inches, at 0.05 psi pressure.

6. The product of any one of claims 1 to 5, having a dry thickness in the range of from about 0.2 inch to about 1 inch, preferably about 0.33 inches to about 0.63 inches, measured at 0.05 psi pressure.

7. The product of any one of claims 1 to 6, wherein the density of said bulk layer is in the range of from about 0.01 to about 0.1 g/cc, preferably about 0.02 to about 0.06 g/cc, most preferably about 0.03 to about 0.05 g/cc.

8. The product of any one of claims 1 to 7, wherein said bulk layer constitutes means for enhancing the resiliency and the stability of said disposable absorbent product.

9. The product of any one of claims 1 to 8, wherein said ultra-thin, functional absorbent core is mounted into an envelope comprising a fluid-permeable cover layer and a fluid-impervious backing layer.

10. The product of claim 9, wherein said bulk layer is placed between said ultra-thin, functional absorbent core and said fluid-impervious backing layer.

11. The product of any one of claims 1 to 10, wherein said bulk layer is made of wood pulp fluff, cotton fibers, rayon fibers, synthetic thermoplastic fibers, a blend of wood pulp and polyethylene fibers, a blend of wood pulp and polyester fibers or a blend of wood pulp and polypropylene fibers.

Fig.3

Fig.2

Fig.1

EP 0 526 225 A1

Fig.6

Fig.5

Fig.4

EP 0 526 225 A1

Fig.9

Fig.8

Fig.7

EP 0 526 225 A1

Fig.12

Fig.11

Fig.10

Fig.13

European Patent Office

**EUROPEAN SEARCH REPORT**

Application Number

EP 92 30 6984

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,P | WO-A-9 111 164 (MOLNLYCKE BV)<br><br>* page 5, line 5 - page 6, line 2; figure 3 *<br>* page 6, line 18 - page 7, line 5 *<br>--- | 1,2,7-9, 11 | A61F13/15 |
| A | EP-A-0 336 578 (THE PROCTER & GAMBLE COMPANY)<br>* abstract *<br>--- | 1-11 | |
| A | EP-A-0 360 680 (KAYSERSBERG SA)<br>* figure 2 *<br>--- | 1-11 | |
| A | NL-A-8 902 635 (T.N.O.)<br>* figures 1,3 *<br><br>----- | 1-11 | |
| | | | **TECHNICAL FIELDS SEARCHED (Int. Cl.5)**<br><br>A61F |

The present search report has been drawn up for all claims

| Place of search | Date of completion of the search | Examiner |
|---|---|---|
| THE HAGUE | 11 NOVEMBER 1992 | ARGENTINI A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)